# EUROPÄISCHE PATENTSCHRIFT

(11) **EP 0 966 921 B1**
(45) Veröffentlichungstag und Bekanntmachung des Hinweises auf die Patenterteilung: **19.09.2001**
(21) Anmeldenummer: 99111621.1
(22) Anmeldetag: 16.06.1999
(51) Int. Cl.: A61B 17/30

(54) **Vorrichtung zum Entfernen von Zecken**
Device for removing ticks
Dispositif pour ôter des tiques

(30) Priorität: 22.06.1998 DE 19827651
(43) Veröffentlichungstag der Anmeldung: 29.12.1999
(73) Patentinhaber: Eckhardt Abform- und Giesstechnik GmbH, 57555 Mudersbach (DE)
(72) Erfinder: Eckhardt, Friedhelm, 57080 Siegen (DE)
(74) Vertreter: Pürckhauer, Rolf, Dipl.-Ing.

(56) Entgegenhaltungen:
- EP-A- 0 159 453
- EP-A- 0 717 963
- WO-A-82/01646
- DE-A- 19 652 218
- US-A- 3 628 824
- US-A- 5 407 243

## Beschreibung

Die Erfindung bezieht sich auf eine Vorrichtung zum Entfernen von Zecken der im Oberbegriff des Patentanspruchs 1 angegebenen Gattung.

Eine solche Vorrichtung in Form einer Kreuzpinzette ist aus EP 0 717 963 A1 bekannt. Bei dieser bekannten Vorrichtung kreuzen sich Greifarme derart, daß Greifbacken unter Vorspannung aneinanderliegen und das auf diese Weise gebildete Maul durch Überwindung der Vorspannung geöffnet werden kann.

Eine ähnliche Kreuzpinzette zum Entfernen von Zecken ist im DE-GM 80 15 364 und in DE 196 52 218 A1 beschrieben.

Die Herstellung und Montage dieser bekannten Vorrichtungen zum Entfernen von Zecken sind relativ kostspielig und umständlich.

Der Erfindung liegt die Aufgabe zugrunde, eine Vorrichtung zum Entfernen von Zecken zu schaffen, die nur aus zwei Kunststoff-Spritzteilen besteht, die leicht und schnell zusammengesetzt werden können.

Diese Aufgabe wird erfindungsgemäß durch die Kennzeichnungsmerkmale des Patentanspruchs 1 gelöst.

Zweckmäßige Weiterbildungen der Erfindung sind den Unteransprüchen zu entnehmen.

Die nur aus zwei Kunststoff-Spritzteilen bestehende erfindungsgemäße Vorrichtung kann leicht und schnell zusammengebaut werden, indem die Greifarme lediglich in das Gehäuse eingeschoben werden, wobei Griffstücke, mit denen die Greifbacken geöffnet werden können, um an einer auf der Haut sitzenden Zecke angesetzt zu werden, in Ausnehmungen des Gehäuses einrasten. Die Greifbacken an den vorderen Enden der Greifarme ragen dann aus einer vorderen Öffnung des Gehäuses heraus und liegen mit einer vorgegebenen Schließkraft aneinander, die durch die Eigenelastizität der aus elastischem Kunststoff einstückig gespritzten Greifarme gegeben ist, deren innere Enden im montierten Zustand der Vorrichtung an Schrägflächen im Gehäuse anliegen und dadurch die Greifarme vorspannen.

Die Vorteile der Erfindung werden klar erkennbar aus der nachfolgenden Beschreibung zweier Ausführungsbeispiele, die in der Zeichnung dargestellt sind. Dabei zeigt bzw. zeigen
- Fig. 1: eine perspektivische Darstellung eines Ausführungsbeispiels der Vorrichtung,
- Fig. 2: eine Umrißansicht eines weiteren Ausführungsbeispiels der Vorrichtung,
- Fig. 3: die Umrißansicht des Ausführungsbeispiels nach Fig. 1,
- Fig. 4: die anfängliche Montierungsphase beim Zusammensetzen der beiden Spritzteile der Vorrichtung,
- Fig. 5: die geöffnete Vorrichtung,
- Fig. 6: die geschlossene Vorrichtung und
- Fig. 7: eine Draufsicht der Greifarme alleine.

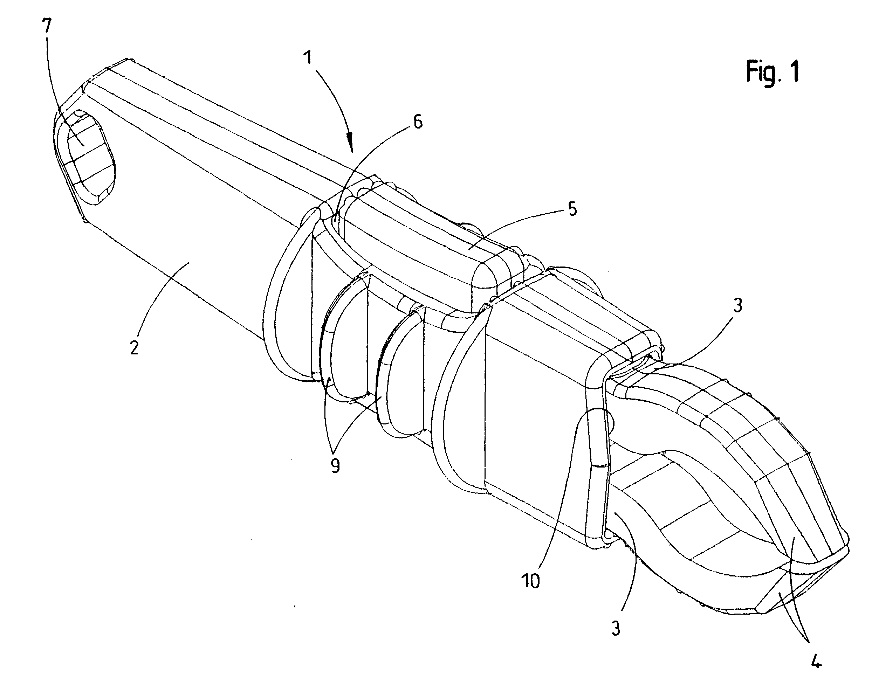

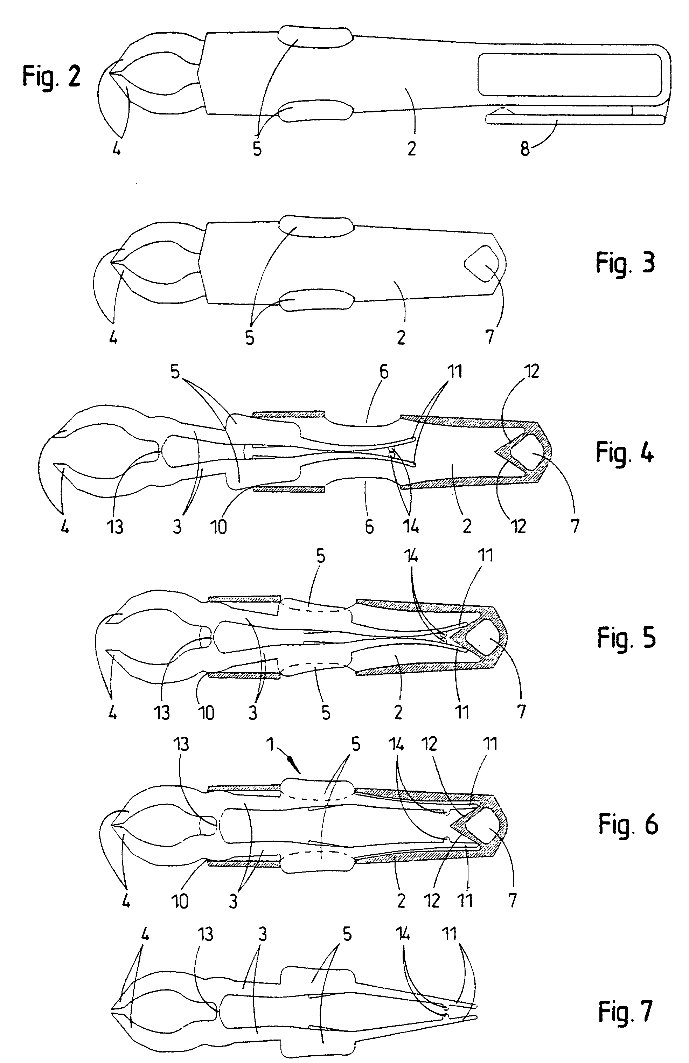

Die Vorrichtung 1 zum Entfernen von Zecken besteht nur aus zwei jeweils einstückig aus Kunststoff gespritzten Teilen, nämlich einem Gehäuse 2, das zweckmäßig aus unelastischem Kunststoff einstückig gespritzt wird, und aus einstückig aus elastischem Kunststoff gespritzten Greifarmen 3. Am vorderen (äußeren) Ende der Greifarme 3 sind Greifbacken 4 angeformt, die aus dem Gehäuse 2 herausragen. Griffstücke 5 sind in entsprechende Öffnungen 6 des Gehäuses 2 eingerastet. Bei dem in Fig. 1 und in den Fig. 3 bis 6 dargestellten Ausführungsbeispiel weist das Gehäuse 2 eine An- oder Aufhängeöse 7 auf, während das in Fig. 2 in Umrissen dargestellte Ausführungsbeispiel der Vorrichtung 1 einen einstückig am Gehäuse 2 angeformten Halteclip 8 aufweist, so daß die Vorrichtung 1 in eine Tasche eines Kleidungsstücks wie z.B. ein Kugelschreiber eingehängt werden kann. Wie Fig. 1 erkennen läßt, weist das Gehäuse 2 in Höhe der Griffstücke 5 seitlich vorstehende runde Rippen 9 auf, so daß die Vorrichtung 1 nach dem Ergreifen einer Zecke, falls nötig, leicht gedreht werden kann.

Die anfängliche Phase beim Zusammensetzen der beiden Teile 2 und 3 der Vorrichtung 1 ist in Fig. 4 dargestellt. Das Gehäuse 2 ist am vorderen Ende offen, und in diese Öffnung 10 des Gehäuses 2 werden die zusammengedrückten Greifarme 3 eingeschoben (Fig. 4), bis die hinteren Enden 11 der Greifarme 3 auf Schrägflächen 12 im Gehäuse 2 auflaufen (Fig. 5). Nach völligem Einschieben der Greifarme 3 in das Gehäuse 2 (Fig. 6) rasten die Griffstücke 5 in die Öffnungen 6 des Gehäuses 2 ein.

Die Greifarme 3 sind über einen Stützsteg 13 miteinander verbunden, der als elastisches Schwenklager zwischen den Greifarmen 3 dient. Damit beim Einschieben der Greifarme 3 in das Gehäuse 2 die inneren Enden 11 der Greifarme 3 sicher auf die Schrägflächen 12 aufgleiten können, sind in einem bestimmten Abstand von diesen inneren oder hinteren Enden 11 Stütznoppen 14 vorgesehen, die beim Zusammendrücken der Greifarme 3, wie aus den Fig. 4 und 5 ersichtlich, die hinteren Enden 11 derselben entsprechend spreizen. Bei völlig eingeschobenen Greifarmen 3 werden diese hinten durch die Schrägflächen 12 abgestützt und so aufgebogen, daß die Greifbacken 4 zusammengedrückt werden, wobei der Stützsteg 13 unter leichter Zugspannung steht.

Zum Gebrauch der Vorrichtung 1 werden die Griffstücke 5 innerhalb der Öffnungen 6 des Gehäuses 2 eingedrückt, so daß sich die Greifbacken 4 öffnen. Die Öffnungsweite kann nach Erfordernis gewählt werden und muß nicht so groß sein wie in Fig. 4 dargestellt, die lediglich die Anfangsphase der Montage zeigt.

In Fig. 7 sind die einstückigen Greifarme 3 noch einmal als Einzelteil in einer Draufsicht bzw. Seitenansicht dargestellt.

Bei gedrückten Griffstücken 5, d.h. bei geöffneter Vorrichtung 1 (Fig. 5) ist zu erkennen, daß die Enden 11 der Greifarme 3, bedingt durch die Verkürzung infolge der Biegung, auf den Schrägflächen 12 nach unten gleiten. Dadurch wird die Vorspannung in den federnden Greifarmen 3 deutlich reduziert, um' eine Überdehnung der Greifarme 3 zu verhindern.

## Patentansprüche

1. Vorrichtung zum Entfernen von Zecken mit zwei teilweise elastischen Greifarmen, an deren freien Enden mit definierter Schließkraft aneinandergedrückte Greifbacken vorgesehen sind, die durch Drücken von an den Greifarmen einstückig angeformten Griffstücken zur Aufnahme einer Zecke zu öffnen sind, **dadurch gekennzeichnet, daß** die Greifarme (3) an einer Stelle zwischen den Griffstücken (5) und den Greifbacken (4) über einen elastischen Stützsteg (13) einstückig miteinander verbunden sind und mit ihren den Greifbacken (4) abgewandten inneren Enden (11) an Schrägflächen (12) anliegen, die im Innern eines die Greifarme (3) aufnehmenden einstückigen Gehäuses (2) an dieses einstückig angeformt sind, wobei die Anordnung so getroffen ist, daß im montierten Zustand der Vorrichtung (1) durch die Anlage der inneren Greifarmenden (11) an den Schrägflächen (12) in Verbindung mit der Eigenelastizität der Greifarme (3) die definierte Schließkraft erzeugt wird.

2. Vorrichtung nach Anspruch 1, **dadurch gekennzeichnet, daß** in einem Abstand von den inneren Enden (11) der Greifarme (3) an diesen einander zugewandte Stütznoppen (14) einstückig angeformt sind, die beim Einschieben der einstückig ausgebildeten Greifarme (3) in das ebenfalls einstückig ausgebildete Gehäuse (2) die inneren Enden (11) der Greifarme (3) spreizen, damit diese auf die Schrägflächen (12) aufgleiten können.

3. Vorrichtung nach Anspruch 1 oder 2, **dadurch gekennzeichnet, daß** die Griffstücke (5) beim völligen Einschieben der Greifarme (3) in das Gehäuse (2) in entsprechende Öffnungen (6) desselben einrasten.

4. Vorrichtung nach einem der vorangehenden Ansprüche, **dadurch gekennzeichnet, daß** die Greifarme (3) aus elastischem Kunststoff einstückig gespritzt sind und daß das Gehäuse (2) aus unelastischem Kunststoff einstückig gespritzt ist.

5. Vorrichtung nach einem der vorangehenden Ansprüche, **dadurch gekennzeichnet, daß** ein Halteclip (8) am Gehäuse (2) einstückig angeformt ist.

6. Vorrichtung nach einem der Ansprüche 1 bis 4, **dadurch gekennzeichnet, daß** das Gehäuse (2) eine An- oder Aufhängeöse (7) aufweist.

7. Vorrichtung nach einem der vorangehenden Ansprüche, **gekennzeichnet durch** in Höhe der Griffstücke (5) seitlich vorstehende runde Rippen (9) am Gehäuse (2).

## Claims

1. Device for removing ticks with two partially flexible gripping arms, on the free ends of which clamping jaws are provided which are pressed against each other with a defined closing pressure and which can be opened in order to receive a tick by pressing grip pieces which are moulded integrally on the gripping arms, **characterised in that** the gripping arms (3) are connected to each other integrally via a flexible support web (13) at a point between the grip pieces (5) and the clamping jaws (4) and abut against diagonal faces (12) by their internal ends (11) which are orientated away from the clamping jaws (4), which diagonal faces are moulded integrally in a housing (2) in the interior of said integral housing which receives the gripping arms (3), the arrangement being such that in a mounted state of the device (1) the defined closing pressure is produced by the abutment of the internal gripping arm ends (11) against the diagonal faces (12) in conjunction with the intrinsic flexibility of the gripping arms (3).

2. Device according to claim 1, **characterised in that**, at a spacing from the internal ends (11) of the gripping arms (3), there are formed integrally on the latter supporting knobs (14) which are orientated towards each other and which spread the internal ends (11) of the gripping arms (3) into the likewise integrally configured housing (2) when the gripping arms (3) which are configured integrally are inserted, in order that said gripping arms can slide along the diagonal faces (12).

3. Device according to claim 1 or 2, **characterised in that** the grip pieces (5), when the gripping arms (3) are completely inserted into the housing (2), lock into corresponding openings (6) of the same.

4. Device according to one of the preceding claims, **characterised in that** the gripping arms (3) are integrally injection-moulded from flexible plastic material and in that the housing (2) is integrally injection-moulded from non-flexible plastic material.

5. Device according to one of the preceding claims, **characterised in that** a holding clip (8) is moulded integrally on the housing (2).

6. Device according to one of the claims 1 to 4, **characterised in that** the housing (2) has an eye (7) for hanging up said device.

7. Device according to one of the preceding claims, **characterised by** round ribs (9) which protrude laterally at the height of the grip pieces (5) on the housing (2).

## Revendications

1. Dispositif pour enlever des tiques, comportant deux bras de préhension partiellement élastiques, aux extrémités libres desquelles sont prévues des mâchoires de préhension qui sont pressées l'une sur l'autre en ayant une force de fermeture définie et qui peuvent être ouvertes pour recevoir une tique en appuyant sur des poignées d'un seul tenant avec les bras de préhension, **caractérisé en ce que** les bras (3) de préhension sont reliés l'un à l'autre d'un seul tenant en un point entre les poignées (5) et les mâchoires (4) de préhension par l'intermédiaire d'une barrette (13) de support élastique et s'applique par leurs extrémités (11) intérieures, éloignées des mâchoires (4) de préhension, à des surfaces (12) inclinées qui sont ménagées d'un seul tenant à l'intérieur d'un boîtier (2) d'un seul tenant recevant les bras (3) de préhension, la disposition étant telle que, lorsque le dispositif (1) est monté, la force de fermeture définie est produite par l'application des extrémités (11) intérieures des bras de préhension aux surfaces (12) inclinées en liaison avec l'élasticité propre des bras (3) de préhension.

2. Dispositif suivant la revendication 1, **caractérisé en ce qu'**il est issu d'un seul tenant des bras (3) de préhension, à une distance des extrémités (11) intérieures de ces bras, des boutons (14) de support qui sont tournés l'un vers l'autre et qui, lorsqu'on enfile les bras (3) de préhension réalisés d'un seul tenant dans le boîtier (2) également réalisé d'un seul tenant, écartent les extrémités (11) intérieures des bras (3) de préhension afin que ceux-ci puissent glisser sur les surfaces (12) inclinées.

3. Dispositif suivant la revendication 1 ou 2, **caractérisé en ce que** les poignées (5) s'encliquètent dans le boîtier (2) dans des ouvertures (6) correspondantes lorsque les bras (3) de préhension sont entièrement enfilés.

4. Dispositif suivant l'une des revendications précédentes, **caractérisé en ce que** les bras (3) de préhension sont obtenus d'un seul tenant par moulage par injection de matière plastique élastique et en ce que le boîtier (2) est obtenu d'un seul tenant par moulage par injection de matière plastique non élastique.

5. Dispositif suivant l'une des revendications précédentes, **caractérisé en ce qu'**un clip (8) de retenue est issu d'un seul tenant du boîtier (2).

6. Dispositif suivant l'une des revendications 1 à 4, **caractérisé en ce que** le boîtier (2) comporte un oeillet (7) d'accrochage ou de suspension.

7. Dispositif suivant l'une des revendications précédentes, **caractérisé par** des nervures (9) rondes en saillie latéralement au niveau des poignées (5) sur le boîtier (2).
